# EUROPEAN PATENT APPLICATION

(11) **EP 2 416 152 A1**
(43) Date of publication of application: **08.02.2012**
(21) Application number: 10171636.3
(22) Date of filing: 02.08.2010
(51) Int. Cl.: G01N 33/26, G01N 33/28, B01L 3/00

(54) **Improvements relating to hydrocarbon recovery**

(71) Applicant: Shell Internationale Research Maatschappij B.V., 2596 HR The Hague (NL)
(72) Inventor: Van Bakel, Martin, 1031 HW Amsterdam (NL); Hoogenboom, Patrick Gerardus Adrianus, 1031 HW Amsterdam (NL); Moene, Robert, 1031 HW Amsterdam (NL); Smit, Jasper Roelf, 1031 HW Amsterdam (NL)
(74) Representative: Matthezing, Robert Maarten

(57) **Abstract**

A method of assessing the hydrocarbon recovery efficacy of a fluid, the method comprising: introducing a hydrocarbon sample into a microchannel region of a microfluidic device; introducing the fluid into the microchannel region to mobilise the hydrocarbon sample; and detecting a distribution of the hydrocarbon sample in the microchannel region with an optical detector. Suitable apparatus are also described.

## Description

### Field of the invention

The present invention relates to a method and apparatus for assessing the hydrocarbon recovery efficacy of fluids, i.e. the ability of fluids to mobilise hydrocarbon deposits.

### Background of the invention

Hydrocarbons are a large class of organic compounds composed of hydrogen and carbon. For example, crude oils, natural gas, kerogen, bitumen, pyrobitumen and asphaltenes are all mixtures of various hydrocarbons. Though hydrocarbons are generally defined as molecules formed primarily of carbon and hydrogen atoms, they may also include other elements, such as, but not limited to, halogens, metallic elements, nitrogen, oxygen and/or sulphur.

Hydrocarbons are typically recovered or produced from subterranean formations by a variety of methods. "Primary recovery techniques" are those techniques that utilise energy from the formation itself to mobilise and recover the hydrocarbons in the subterranean formation. However primary recovery techniques are only capable of producing a small fraction of the oil in place in the reservoir. Consequently secondary and tertiary recovery techniques have been developed, which have as their primary purpose the mobilisation and recovery of additional quantities of hydrocarbons known to be present in the reservoir.

The secondary technique of water-flooding employs water as a hydrocarbon recovery fluid, by injecting it into hydrocarbon formations to enhance production. However, waterflooding is of limited effectiveness, primarily due to the generally high surface tension between water and hydrocarbons, which is a barrier to mobilisation.

In certain tertiary hydrocarbon recovery techniques, commonly referred to as "chemical enhanced oil recovery" (cEOR), surface-active agents, polymers, alkalis, salts and other chemicals are mixed with water to form improved hydrocarbon recovery fluids. These improved fluids lead, for example, to reduced surface tension and better hydrodynamic properties, thereby enhancing hydrocarbon mobilisation and recovery.

There are many patents that describe cEOR utilizing chemical surfactants and polymers, including among others U.S. Pat. Nos. 3,508,611, 3,823,777, 3,981,361, 4,058,467, 4,203,491, 4,232,738, 4,362,212, 4,411,816, 4,458,755, 4,493,371, 4,501,675, 4,502,541, 4,799,547, 5,031,698, 5,068,043, 6,022,834, 6,613,720, 6,989,355 and Canadian Pat. No. 1,169,759.

There is an ongoing need for improved hydrocarbon recovery fluids. Accordingly, it is desirable to test the hydrocarbon recovery efficacy of a great number of fluids, i.e. their ability to mobilise hydrocarbons. Notably, conditions within hydrocarbon formations can vary significantly, both geographically and over time, which means that hydrocarbon recovery efficacy tests often needs to be carried out on a per-formation basis. This further increases the requisite volume of testing.

Prior art laboratory techniques for assessing the hydrocarbon recovery efficacy of fluids tend either to lack realism or be overly expensive and time-consuming.

For example, one such technique involves observing, within a glass tube, the tendency of fluids to form microemulsions with hydrocarbon samples, at varying salinities. However, this technique provides only a rough indication of hydrocarbon recovery efficacy because a glass tube cannot accurately simulate the porous rock environments typically encountered in hydrocarbon formations. Furthermore, this technique is suitable only for studying surface tension, and provides little or no information on hydrodynamic effects such as sweep and fingering, which can also have a significant impact on hydrocarbon mobilisation.

A second technique, which aims to provide a more realistic emulation of the conditions within a hydrocarbon formation, involves driving hydrocarbon (e.g. oil) and a recovery fluid through rock core samples obtained from a given hydrocarbon formation. This technique can provide a good indication of the hydrocarbons recovery efficacy of a given fluid in a given environment. However, it is costly and time-consuming to implement, and can hence only be contemplated for a small selection of hydrocarbon recovery fluids.

Against this background, the technical problem underlying the present invention is to provide a method and apparatus for assessing the hydrocarbon recovery efficacy of a given fluid accurately but yet cost-effectively and in a scalable manner.

### Summary of the invention

From a first aspect, the invention broadly resides in a method of assessing the hydrocarbon recovery efficacy of a fluid, the method comprising: a)introducing a hydrocarbon sample into a microchannel region of a microfluidic device; b) introducing the fluid into the microchannel region to mobilise the hydrocarbon sample; and c) detecting a distribution of the hydrocarbon sample in the microchannel region with an optical detector.

The method of the invention provides accurate results, but is yet cost-effective and scalable. On the one hand, the microchannel region, which may be made of glass or other transparent material and may comprise a plurality of interconnected microchannels, provides a reasonable emulation of porous rock. On the other hand, the detection of a hydrocarbon distribution with an optical detector represents a convenient and readily scalable measurement of hydrocarbon recovery efficacy. The method of the invention thus enables the effective assessment (or characterisation) of a large number of prospective hydrocarbon recovery fluids.

The introduction of the hydrocarbon sample and the fluid into the microchannel region may occur in any given order or simultaneously. However, preferably, the hydrocarbon sample may be introduced before the fluid is introduced. Optionally, the method may comprise introducing an aqueous phase into the microchannel region, e.g. after the hydrocarbon sample is introduced and before the fluid is introduced. This mimics waterflooding conditions encountered in hydrocarbon formations. The aqueous phase may simply comprise water, optionally with dissolved components such as salt.

To emulate hydrocarbon recovery conditions particularly accurately, the method may comprise flooding the microchannel region with the hydrocarbon sample, optionally introducing an aqueous phase into the microchannel region, and thereafter driving the fluid into the microchannel region. Conveniently, the hydrocarbon sample and/or the fluid and/or the aqueous phase may be introduced or driven into the microchannel region under pressure.

Detection of the distribution of the hydrocarbon sample following the introduction of both the hydrocarbon sample and the liquid may be based on detecting a discontinuity or contrast between the hydrocarbon sample and the fluid. This continuity or contrast may occur naturally, or may be created by dyeing the hydrocarbon sample and/or the fluid. Whilst detection of the distribution of the hydrocarbon sample is generally the most efficient way to assess the hydrocarbon recovery efficacy of the fluid, it is optionally contemplated by the invention to detect the distribution of the fluid, either instead of or in addition to the distribution of the hydrocarbon sample. Detecting the distribution of the fluid allows inferences to be made about the distribution of the hydrocarbon sample, and accordingly any references herein to the detection of the hydrocarbon sample are to be read as optionally encompassing the detection of the fluid, either additionally or alternatively.

The detection of the distribution of the hydrocarbon sample may provide a characterisation of an interaction between the hydrocarbon sample and the fluid. Such a characterisation, and by extension the assessment of the hydrocarbon efficacy of the fluid, may be based on a single detected distribution, or a comparison of a plurality of detected distributions of the hydrocarbon sample.

For example, the method may comprise determining a degree of mobilisation of the hydrocarbon sample by comparing a pre-mobilisation distribution (or amount) of the hydrocarbon sample in the microfluidic region detected before the introduction of the fluid, with a post-mobilisation distribution (or amount) of the hydrocarbon sample in the microfluidic region detected after the introduction of the fluid. The post-mobilisation distribution may for example be detected after a predetermined time, or after the introduction of a predetermined amount of the fluid. The degree of mobilisation of the hydrocarbon sample determined in this manner is an indicator of the hydrocarbon recovery efficacy of the fluid: the greater the degree of mobilisation, the higher the efficacy of the fluid.

Advantageously, the method may also provide for the detection and observation of dynamic effects such as fingering, sweep and microemulsion formation, which occur during mobilisation of the hydrocarbons sample by the fluid. The detection of such effects enables additional characterisation of the fluid, which may be taken into account in assessing the hydrocarbon recovery efficacy of the fluid. Thus the method may preferably comprise detecting one or more distributions of the hydrocarbon sample in the microchannel region during mobilisation of the hydrocarbon sample by the fluid, optionally to record a hydrodynamic effect.

It has been found that where a microemulsion is formed by the fluid and the hydrocarbon sample, it may be possible to detect the distribution of the microemulsion. The distribution or extent of the microemulsion is a direct indicator of the hydrocarbon recovery efficacy of the fluid: usually, there is a direct relationship between the amount of microemulsion formed and the efficacy of the fluid. Accordingly, the step of detecting a distribution of the hydrocarbon sample in the microchannel region with an optical detector may comprise or consist of detecting, with the optical detector, the distribution of a microemulsion formed by the fluid and the hydrocarbon sample.

To capture maximum information about the fluid, particularly in relation to dynamic effects, the method may preferably comprise detecting the distribution of the hydrocarbon sample over substantially the entire microchannel region. However, it is also possible to assess the hydrocarbon recovery efficacy of a fluid by detecting the hydrocarbon distribution in only a part of the microchannel region.

A further factor that contributes towards capturing maximum information about the fluid is the resolution of the optical detector. Preferably, the distribution of the hydrocarbon sample may be detected at a high resolution, e.g. at least 1000 PPI (pixels per inch), preferably at least 3000 PPI, most preferably at least 6000 PPI. A high resolution is particularly advantageous where the microchannel region comprises microchannels with a small diameter, e.g. below 50 µm. Most preferably, the method may comprise detecting the distribution of the hydrocarbon sample over substantially the entire microchannel region at a high resolution, as this provides information both on a macroscopic scale, (e.g. inch dimensions) and on a microscopic, pore-level scale (e.g. micrometer dimensions), enabling an extensive assessment of the hydrocarbon recovery efficacy of the fluid.

To further improve scalability of the method of the invention, at least one distribution of the hydrocarbon sample may be detected in parallel (i.e. simultaneously) with that of at least one other hydrocarbon sample in at least one other microfluidic device. This conveniently increases the speed at which a large number of fluids can be assessed.

To further facilitate the assessment of fluids, the method may comprise logging at least one distribution of the hydrocarbon sample using a computing device operably connected to the optical device.

The logging of hydrocarbon sample distributions in a computing device enables an automation of the assessment of the hydrocarbon recovery fluids. For example, the method of the invention may comprise applying pattern recognition software to an image of a hydrocarbon sample distribution detected by the optical device in order to express the distribution (or detected amount of hydrocarbon sample) as a numerical value. Converting the distribution into a value, enables an automated assessment or measurement of the hydrocarbon recovery efficacy of the fluid. For example mobilisation of the hydrocarbon sample, which represents a direct measurement of the hydrocarbon recovery efficacy of the fluid, may be expressed numerically, as the difference between a first value representing a pre-mobilisation hydrocarbon sample distribution (or amount) in the microchannel region and a second value representing a post-mobilisation hydrocarbon sample distribution (or amount) in the microchannel region. In more general terms, the method may comprise tracking a plurality of distributions of the hydrocarbon sample in the microchannel region over time to provide a hydrocarbon mobilisation profile of the fluid.

Whilst it is possible to assign a value to the mobilisation of the hydrocarbon sample, and by extension to the hydrocarbon recovery efficacy of the fluid, the method also contemplates assessing the efficacy of the fluid relative to that of another fluid. This may conveniently be achieved by carrying out the method of the invention on a plurality of fluids in parallel and comparing comparable detected distributions of the hydrocarbon samples. Alternatively, particularly where hydrocarbon sample distributions are logged and/or expressed as numerical values, relative assessment may conveniently be carried out nonconcurrently, e.g. by reference to a scale or database. This is of great benefit when assessing a large number of fluids.

The fluid may preferably be a liquid. However, it is also possible to use gasses such as carbon dioxide as hydrocarbon recovery fluids and thus as fluids in the method of the invention.

To prevent excessive pressure build-up within the microchannel region, the method may conveniently comprise draining the microchannel region. The microchannel region may preferably be drained before the distribution of the hydrocarbon sample is detected, as the hydrocarbon sample distribution remaining after draining the microchannel region may be indicative of the mobilisation of the hydrocarbon sample. Most conveniently, the microchannel region may be drained continuously, for example by leaving open an outlet of the microchannel region.

From a second aspect, the invention broadly resides in an apparatus (or set or system) for determining the hydrocarbon recovery efficacy of a fluid, the apparatus comprising: a microfluidic device comprising a microchannel region and an inlet for introducing hydrocarbon samples and fluids into the microchannel region; and an optical detector for detecting the distribution of hydrocarbon in the microchannel region.

The apparatus may be used to carry out the method of the first aspect of the invention, and the advantages described in respect of the method thus apply mutatis mutandis to the apparatus. Accordingly, the apparatus may comprise means for carrying out the optional and preferred features described above in respect of the method. Likewise, the method may comprise steps that make use of the preferred features of the apparatus.

The microfluidic device, and in particular its microchannel region, may be comprised of glass or any other material suitable for allowing the detector to detect the distribution of hydrocarbon within the microchannel region (e.g. polymers such as polydimethylsiloxane). The surface of the microchannel region may be hydrophilic or hydrophobic, as desired to assess the fluid.

The microchannel region of the or each microfluidic device of the apparatus may advantageously comprise a plurality of interconnected microchannels, preferably formed by an etching process. The alignment of the microchannels may be irregular. Alternatively, the microchannel region may comprise a first set of substantially parallel microchannels and a second set of substantially parallel microchannels intersecting the microchannels of the first set at a plurality of junctions. The microchannels of the first set may be substantially orthogonal to the microchannels of the second set.

Preferably, the microchannel region may comprise a single plane of microchannels. Alternatively, the microchannel region may optionally comprise a plurality of interconnected planes of microchannels.

The diameter of the microchannels may advantageously be at most 100 µm, preferably at most 50 µm, more preferably at most 40 µm, and most preferably at most 30 µm. The diameter of the microchannels may be uniform. Alternatively, the diameter may vary to mimic rock pore structures.

Advantageously, the microchannels may comprise one or more surface-increasing formations, such as spurs. The surface-increasing formations, which may preferably be blind and/or generally orthogonal with respect to an associated microchannel, serve to trap hydrocarbon in the manner of pores in hydrocarbon-bearing rock. A microchannel region comprising surface-increasing formations can thus provide an even more accurate emulation of a hydrocarbon production environment. Notably, blind formations oriented generally orthogonally with respect to associated microchannels, provide a good mobilisation challenge for fluid introduced into the microchannel region.

The microchannel region may be of any size and shape, but is preferably oblong. To enable accurate detection by the optical detector, the microchannel region, and optionally the microfluidic device as a whole, may preferably have a thickness of less than 10 mm to facilitate alignment with the focal length of the optical device. Furthermore, a microchannel region footprint in the range of from 15 cm² to 80 cm², preferably 30 cm² to 50 cm² has been found to offer a good balance between handling convenience and emulation accuracy.

The optical detector may be of any type suitable for detecting the distribution of hydrocarbon within the microchannel region. However, the optical detector may preferably be arranged to support the microchannel region of the microfluidic device, or optionally the microfluidic device as a whole, on a detection bed or surface. Preferably, a focal point of the optical device may lie beyond or above the detection bed or surface, e.g. within the microfluidic device supported by the detection bed in use.

To capture maximum information about the fluid, particularly in relation to dynamic effects, the detector may preferably be arranged to detect the distribution of the hydrocarbon sample over substantially the entire microchannel region. Additionally or alternatively, the detector may be capable of operating at a high resolution, e.g. at least 1000 PPI (pixels per inch), preferably at least 3000 PPI, most preferably at least 6000 PPI.

To enable the assessment of a large number of fluids, the apparatus may preferably comprise a plurality of microfluidic devices. The optical detector may then be arranged to support and detect the distribution of hydrocarbon in a plurality of microchannel regions or microfluidic devices in parallel (i.e. simultaneously).

Conveniently, for example to achieve the preferred advantages set out above, the optical detector may be a scanner, preferably a flat-bed scanner. The detector may preferably be arranged to detect the hydrocarbon sample with one or more passes of a detector array, as this enables advantageously high resolutions.

The apparatus may further comprise a computing device operably connected to the optical device, the computing device being arranged to log the distribution of hydrocarbon in the microchannel region. Preferably, the computing device may be arranged to express a distribution or an amount of hydrocarbon detected by the optical detector in the microchannel region as a numerical value. For example, the computing device may comprise pattern recognition software. The computing device may be arranged to control the operation of the apparatus as a whole, and may comprise a user interface for receiving instructions from a user.

The apparatus may comprise a test control module for controlling test conditions of the apparatus. The test module may advantageously comprise a heating and/or cooling device arranged to control the temperature of the fluid and hydrocarbon samples introduced into and within the microfluidic device. For example, the heating device may comprise a transparent temperature control panel, e.g. electrically heated glass, suitable for being brought into contact with the microfluidic device in use without preventing detection of the hydrocarbon by the detector. Thus, advantageously, the detector may be arranged to detect the hydrocarbon through the temperature control panel.

To facilitate the introduction of fluids into the microchannel region, the apparatus may comprise a driver for driving fluid and/or hydrocarbon samples, and/or optionally an intermediate aqueous phase, into, and preferably through, the microchannel region. The driver may preferably comprise at least one syringe. To prevent excessive build-up of pressure, the microfluidic device may comprise an outlet linked to the microchannel region.

From a third aspect, the invention resides broadly in an optical detector for detecting the distribution of hydrocarbon in a microfluidic device, the detector comprising: detection means having a focal point; and a detector surface or bed for supporting the microfluidic device, wherein the focal point is arranged to lie beyond or above the detection bed, e.g. within a microfluidic device supported on the detector surface in use.

The preferred features recited in respect of the optical detector of the apparatus according to the second aspect of the invention may also be applied to the optical detector according to the third aspect of the invention.

Further optional features and advantages of the various aspects of the invention will be apparent from the following detailed description of the invention.

### Detailed description of the invention

In order that the invention may be more readily understood, reference will now be made, by way of example, to the accompanying drawings in which:
Figure 1 is a schematic view of an apparatus for assessing the hydrocarbon recovery efficacy of fluids in accordance with an exemplary embodiment of the present invention;
Figure 2 is a planar, view of a microfluidic device of the apparatus of Figure 1, with two progressive, partial magnifications;
Figures 3a to 3d are sequential enlarged views of an area of the microchannel region of the microfluidic device of Figure 2, detected during the assessment of the hydrocarbon recovery efficacy of a fluid; and
Figures 4a to 4d are the same views as Figures 3a to 3d, shaded to highlight a hydrocarbon sample.

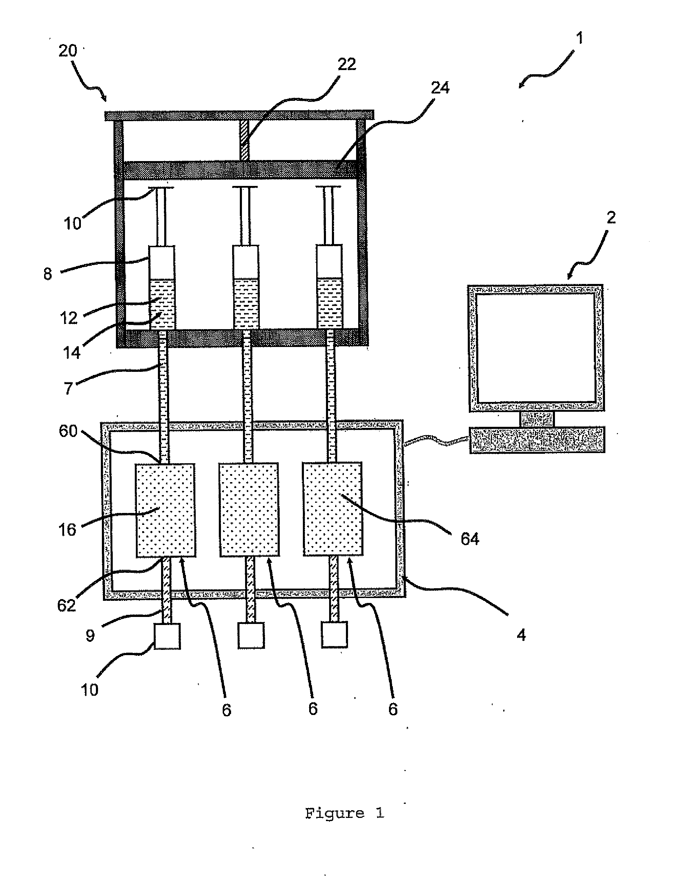

Referring firstly to Figure 1, an apparatus 1 for assessing the hydrocarbon recovery efficacy of fluids comprises an array of three identical microfluidic devices 6 and an optical detector in the form of a modified flatbed scanner 4.

The microfluidic devices 6 each comprise a microchannel region 64 and an inlet 60 for introducing samples and fluids into the microchannel region 64 with the help of syringes 8 and a pump 20.

Each syringe 8 comprises a fluid reservoir 12, and a plunger 10 that can be actuated to drive fluid, such as a prospective hydrocarbon (e.g. oil) recovery fluid 14 within the fluid reservoir 12, out of the syringe 8, and via an inlet pipe 7 to the inlet 60 of its associated microfluidic device 6. To prevent excessive pressure build-up within the microfluidic devices 6, an outlet 62 of each microfluidic device 6 is connected via an outlet pipe 9 to a drain 10.

The pump 20 comprises a plunger bar 24 the position of which is controlled by a screw-thread drive 22. The pump 20 holds the syringes 8 in position, and by virtue of the movement of the plunger bar 24 can simultaneously actuate the plungers 10 of each syringe 8.

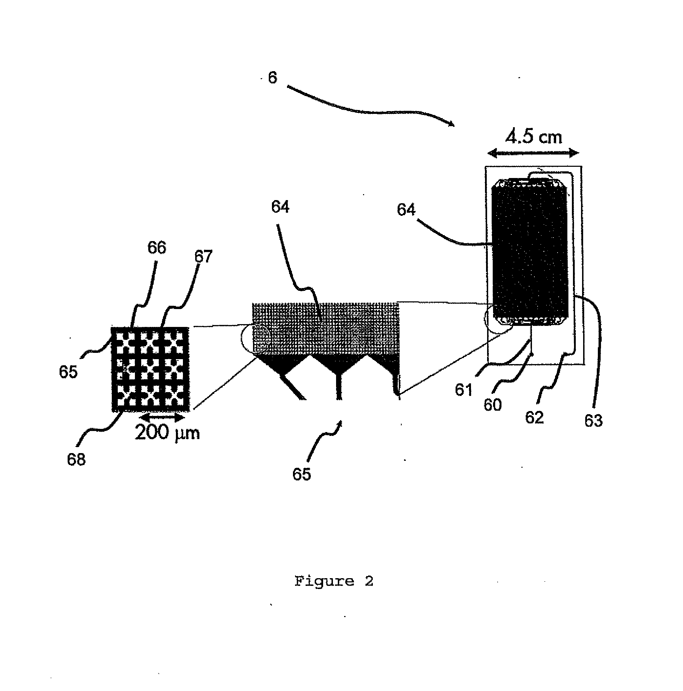

Referring now to Figure 2, each of the microfluidic devices 6 is oblong with a width of approximately 4.5 cm, a length of approximately 9 cm and a depth of a few millimetres.

As aforesaid, the microfluidic devices 6 each comprise a microchannel region 64, which is also roughly oblong and extends partway along the length of the microfluidic device 6 between an inlet channel 61 and an outlet channel 63. The inlet channel 61 and the outlet channel 63 meet the microchannel region 64 at opposite ends thereof, at respective branched junctions 65. The inlet junction 65 allows fluid flowing from the single inlet channel 61 to be spread along the width of one end of the microchannel region 64 prior to entering the microchannel region 64. Similarly, fluid emerging from the microchannel region 64 at the other end is funnelled to join into the single outlet channel 63. Thus even delivery of fluid samples to/from entire microchannel region 64 is achieved.

The inlet channel 61 of each microfluidic device 6 is routed to the inlet 60 of the device, whilst the outlet channel 63 is routed to the associated outlet 62. Functionally, the arrangement of the channels 61, 63 and the microchannel region 64 is such that the microfluidic device 6 is bidirectional, in that the inlet could be used as an outlet and vice-versa, if desired.

The microchannel region 64 comprises a plurality of interconnected microchannels. A first set of parallel microchannels 65 runs longitudinally aligned with the length of the microfluidic device 6 and a second set of parallel microchannels 66 runs laterally aligned with the width of the microfluidic device 6, within the same plane. Where the first and second set of channels meet, they form network junctions 67. The spacing between adjacent parallel microchannels 64, 65 is approximately 100µm (100 micrometers) and the approximate diameter of each microchannel is 30µm.

Along each of the first and second microchannels, 65, 66, between each of the network junctions 67, are pairs of spurs 68 branching orthogonally away from their respective channels 65, 66. For example, midway between two adjacent network junctions 67 on a longitudinally-running network channel are two spurs 68 extending from the channel, away from one another in the lateral direction. Each spur 68 is blind and so only connects with the network channel from which it branches.

The combination of fluid channels 65, 66, junctions 67 and spurs 68 is suitable for emulating capillary phenomena that may be found in porous rock. It will be noted that the junctions 67 and in particular the spurs 68 create a fluid path of non-uniform cross-section that presents fluids flowing through the capillary network with a large number of cells into which the fluids may become trapped. This is a useful feature when attempting to test the hydrocarbons recovery efficacy of fluids 14, i.e. the capability of fluids to mobilise hydrocarbons.

The microchannels, 65, 66, the network junctions 67 and the spurs 68 are all in fluid communication to one another to define the microchannel region 64.

Referring again to Figure 1, the microchannel regions 64 of the microfluidic devices 6 rest on a scanning or detection surface/bed of the scanner 4 and are made of substantially transparent glass or other transparent material like polymers, e.g. PDMS, so that the flow of fluid and hydrocarbon within them is visible from their exterior. The flatbed scanner 4 is of conventional type, though its focal point has been shifted to lie just above the detection surface, within the microfluidic devices resting thereon. Therefore, the scanner 4 can be used to capture images of the interior of each microfluidic device 6 and in particular to detect the distribution and behaviour of any fluids within the microchannel regions 64. Notably, the array of microfluidic devices 6 resting on the scanner can be scanned in parallel, i.e. substantially simultaneously.

The flatbed scanner 4 can provide higher resolutions than other optical detectors such as digital cameras. This is as a result of the structure and arrangement of the components of flatbed scanners which is well-known in the art.

The detection surface of the scanner 4 is illuminated by a bright light-source (not shown), which is aligned with a moving optical array (not shown). The optical array, which may comprise Charge Coupled Detectors (CCD) or Contact Image Sensors (CIS), passes across the detection surface together with the light source, reading the entire area.

As the microfluidic devices 6 rest on the detection surface, their position relative to the optical array is predictable and in close proximity. Hence, the scanner is able to capture images at a high resolution (e.g. 3000 PPI) using one or more passes of the optical array.

The flatbed scanner 4 is operably connected to a computing device 2 which is arranged to issue commands to the scanner 4 to conduct a scanning operation, and receives images from the scanner 4 as a result of a scanning operation. The computing device 2 is also arranged to store and process images of each microfluidic device 6 over time to record the flow behaviour of fluid passing through each microfluidic device 6. The computing device 2 is arranged to receive inputs about the experimental conditions of the apparatus. For example, the computing device may receive inputs about the rate at which fluid is pumped to microfluidic devices, temperature, pressure, the type of fluid being pumped, the type of microfluidic device being used and so forth. The inputs and images received are matched and catalogued by the computing device 2 to allow for viewing and/or analysis. Furthermore, the computing device 2 is equipped with pattern recognition software.

Referring still to Figure 1, in use, the apparatus 1 functions to assess the hydrocarbon recovery efficacy of fluids. A plurality of fluids can be tested and compared in parallel, for example to determine their relative efficacy on a given hydrocarbon sample.

To prime the apparatus 1, each of the microfluidic devices 6 is flooded with a hydrocarbon sample 16, such as Brent Crude. This is achieved by manually connecting each microfluidic device 6 to a priming syringe (not shown) containing the hydrocarbon sample 16, and then injecting the sample 16 into each microfluidic device 6 from the priming syringe. Once flooded with hydrocarbon 16, each microfluidic device 6 is disconnected from the priming syringe and placed onto the detection surface of the scanner 4. The microfluidic devices 6 are held in place on the scanner 4 using adhesive so as to prevent their accidental movement during the test.

Once securely in place, each microfluidic device 6 is coupled to its associated syringe 8 containing oil recovery fluid 14 to be tested. The pump 20 is then initialised to drive oil recovery fluid 14 simultaneously from each of the syringes 8 into each respective microfluidic device 6. On initialisation of the pump 20, the computing device 2 is also instructed to operate the scanner 4 to capture at intervals images of the microchannel region of each microfluidic device 6. The images captured at intervals are then stored and processed by the computing device 2 to build up a series of images, or a video, depicting the flow behaviour of fluids being pumped through each of the microfluidic devices 6 over time, and in particular the distribution and mobilisation of the hydrocarbon sample 16 in each device 6. Typically there is an optical contrast between hydrocarbon samples and recovery fluids. However, where a sufficient contrast is absent, the sample and/or the recovery fluid may be dyed as necessary.

One way of analysing the images detected by the scanner 4 and stored in the computing device 2 is to carry out a hydrocarbon distribution comparison with comparable stored images detected in respect of other fluids, e.g. using pattern recognition software. This provides a relative indication of hydrocarbon recovery efficacy. Alternatively, hydrocarbon distribution or prevalence may be rated on a pre-defined scale.

As oil recovery fluid 14 is pumped into each of the microfluidic devices 6, it interacts dynamically with the hydrocarbon sample 16 therein. For example, certain suitably surface active fluids may form a microemulsion with the hydrocarbon sample 16. In general, the formation of a stable microemulsion can reduce capillary forces, thereby enhancing mobility and hydrocarbon recovery. In some cases this microemulsion shows up as a separate phase (with a different colour) detectable by the scanner 4. The extent of microemulsion formation can be used as an indicator of the hydrocarbon recovery efficacy of the fluid, and may thus be recorded and processed by the computing device 2 as described above. Similarly, the scanner 4 may detect other phenomena such as fingering and sweep, which can be stored, processed and analysed by the computing device 2, for example by comparison with results from other fluids.

If the hydrocarbon sample 16 is not sufficiently mobilised then pockets of oil can remain trapped within the microchannel region 64, especially between junctions 67 and within spurs 68 where walls of the microchannel region 64 present the oil with the largest surface area against which to adhere. The extent to which hydrocarbon is mobilised, and hence the hydrocarbon efficacy of the fluid 14, can be represented by the distribution or quantity of oil remaining trapped within the microchannel region 64 after a given injection of fluid 14.

Although the microfluidic devices 6 may not faithfully represent the structure of porous rock, they represent a reasonable emulation. Furthermore, the apparatus 1 provides a cost-effective and scalable solution for determining the hydrocarbon recovery efficacy of fluids. In particular, a large number of fluids can be rigorously tested and compared on a standardised basis using the apparatus 1.

It will be understood that many variations and modifications to the apparatus according to the present embodiment are possible.

In alternatives, the computing device may be operably connected to a test control module that is arranged to control the test conditions of the apparatus. For example, the test module may comprise a heating and/or cooling device arranged to control the temperature of the fluid samples introduced into and within the microfluidic devices.

In alternatives, the apparatus 1 may comprise more than three microfluidic devices - for example, nine microfluidic devices having a footprint size of 9 cm x 4.5 cm can be easily accommodated on a standard flatbed A4 scanner.

### Example of the invention

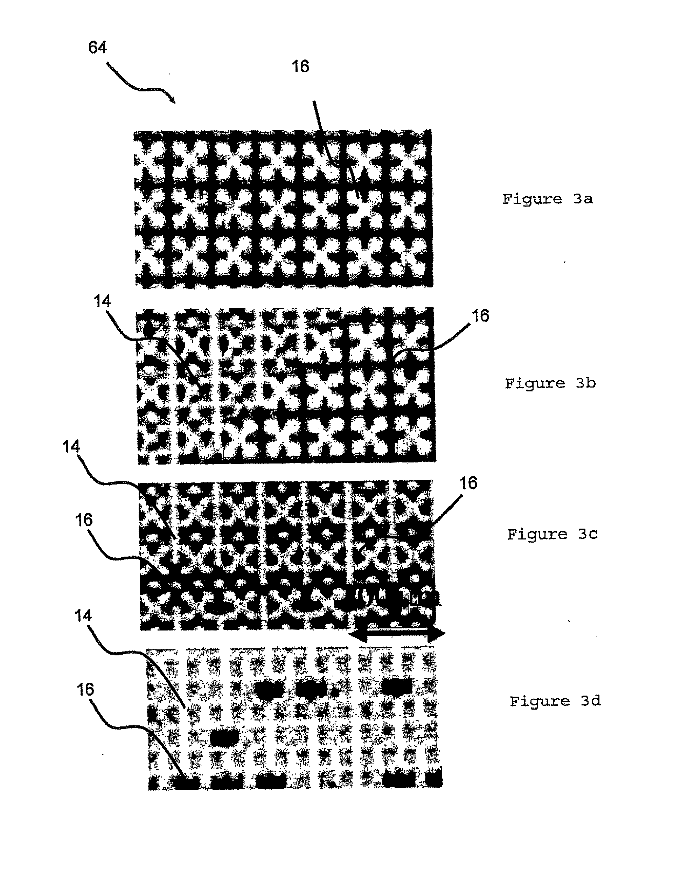

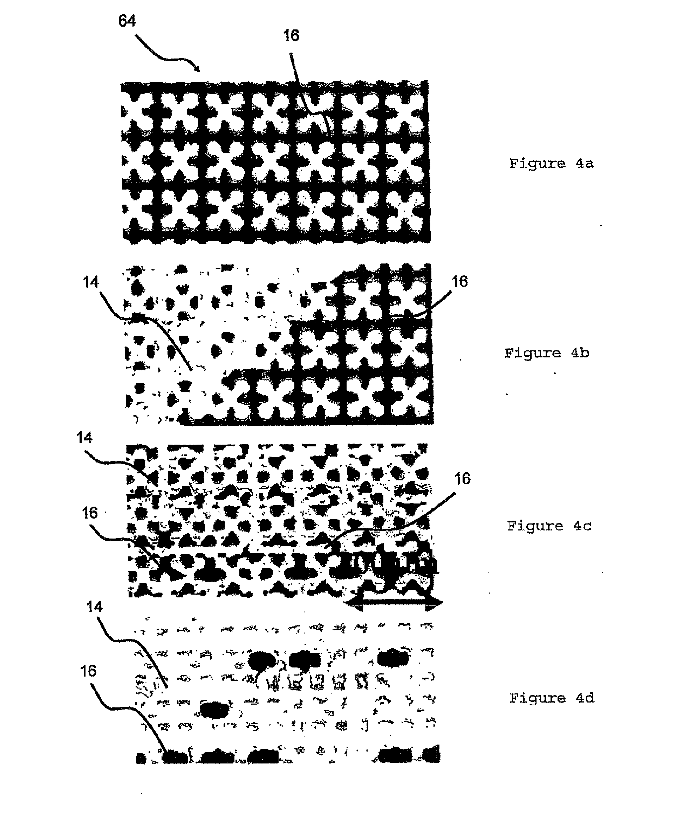

To illustrate the working of the apparatus 1 according to the embodiment above, reference is now made to Figures 3a to 3d, and 4a to 4d.

Figures 3a to 3d are unmodified sequential images of an area of a microchannel region 64 captured by the scanner 4 according to the embodiment described above. In Figures 4a to 4d the same images have been shaded to highlight the presence of hydrocarbons, and thus help visualise hydrocarbon mobilisation.

Figures 3a and 4a show the microchannel region 64 in a first stage, completely flooded with a hydrocarbons sample 16, specifically Brent Crude.

Figures 3b and 4b show the microchannel region 64 during a first test, in which an aqueous solution comprising 13% (wt) NaCl, is driven through the microfluidic device 6.

Figures 3c and 4c show a part of the microchannel region 64 of Figures 3b and 4b after the first test, i.e. after NaCl solution has been pumped through it until mobilisation of oil has essentially stopped.

For comparison, Figures 3d and 4d show the same part of the microchannel region 64 after a second test in which, following the first test, a predetermined quantity of a second aqueous solution comprising surfactant is driven through the microfluidic device. The second aqueous solution comprises 0.5w% Enordet® 0332 surfactant, 13w% NaCl and SBA (sec-butanol).

As can be seen by comparing Figures 3c and 4c against Figures 3d and 4d, an aqueous solution containing surfactants results in further mobilisation of the oil (Figures 3d and 4d) than was achievable with a so-called "water-flood" of salt water (Figures 3c and 4c), as fewer pockets of oil remain trapped within the microchannel region 64. Whilst this result is not surprising, it demonstrates that the present invention is able to assess accurately and effectively the hydrocarbon recovery efficacy of fluids, and readily enables an efficacy comparison between a plurality of fluids.

Furthermore, it is noted that a sequence of images (or a video) captured via the scanner 4 by the computing device 2 for each microfluidic device 6 can be analysed to determine quantitatively not only the overall efficacy of fluids under test (e.g. by counting the number of remaining oil pockets per unit area or counting the number of pixels in an image corresponding to hydrocarbon), but also the flow behaviour of the fluid samples over time.

Contrary to prior art techniques, the present invention thus provides in-situ analysis of fluids in the context of hydrocarbon recovery efficacy, both on a microscopic and a macroscopic scale. This can provide valuable insights, for example into hydrodynamic effects, and the reasons for the degree of hydrocarbon recovery efficacy of a given fluid.

## Claims

1. A method of assessing the hydrocarbon recovery efficacy of a fluid, the method comprising:
a) introducing a hydrocarbon sample into a microchannel region of a microfluidic device;
b) introducing the fluid into the microchannel region to mobilise the hydrocarbon sample; and
c) detecting a distribution of the hydrocarbon sample in the microchannel region with an optical detector.

2. The method of claim 1 comprising flooding the microchannel region with the hydrocarbon sample, introducing an aqueous phase into the microchannel region to mimic a waterflood, and thereafter driving the fluid into the microchannel region.

3. The method of any preceding claim, comprising determining a degree of mobilisation of the hydrocarbon sample by comparing a pre-mobilisation distribution of the hydrocarbon sample in the microfluidic region detected before the introduction of the fluid, with a post-mobilisation distribution of the hydrocarbon sample in the microfluidic region detected after the introduction of the fluid.

4. The method of any preceding claim comprising detecting one or more distributions of the hydrocarbon sample in the microchannel region during mobilisation of the hydrocarbon sample by the fluid.

5. The method of any preceding claim comprising detecting the distribution of the hydrocarbon sample over substantially the entire microchannel region at a resolution of at least 1000 PPI.

6. The method of any preceding claim, wherein the distribution of the hydrocarbon sample is detected in parallel with that of at least one other hydrocarbon sample in at least one other microfluidic device.

7. The method of any preceding claim comprising logging the distribution of the hydrocarbon sample using a computing device operably connected to the optical device.

8. The method of any preceding claim comprising tracking a plurality of distributions of the hydrocarbon sample in the microchannel region over time to provide a hydrocarbon mobilisation profile of the fluid.

9. The method of any preceding claim, wherein the hydrocarbon recovery efficacy of the fluid is assessed relative to that of another fluid.

10. Apparatus for determining the hydrocarbon recovery efficacy of a fluid, the apparatus comprising:
a microfluidic device comprising a microchannel region and an inlet for introducing hydrocarbon samples and fluids into the microchannel region; and
an optical detector for detecting the distribution of hydrocarbon in the microchannel region.

11. The apparatus of claim 10, wherein the microchannel region comprises a plurality of interconnected microchannels comprising surface-increasing formations.

12. The apparatus of claim 10 or claim 11, wherein the optical detector is arranged to support the microchannel region of the microfluidic device on a detection bed.

13. The apparatus of any one of claims 10 to 12 wherein the optical detector is arranged to support, and detect the distribution of hydrocarbon in, a plurality of microchannel regions in parallel.

14. The apparatus of any one of claims 10 to 13 further comprising a computing device operably connected to the optical device, the computing device being arranged to log a detected distribution of hydrocarbon in the microchannel region.

15. An optical detector for detecting the distribution of hydrocarbon in a microfluidic device, the detector comprising:
detection means having a focal point; and
a detection bed for supporting the microfluidic device,
**characterised in that** the focal point is arranged to lie above the detection bed, within a microfluidic device supported on the detection bed in use.
